# EUROPÄISCHE PATENTANMELDUNG

(11) **EP 3 095 775 A1**
(43) Veröffentlichungstag der Anmeldung: **23.11.2016**
(21) Anmeldenummer: 15168364.6
(22) Anmeldetag: 20.05.2015
(51) Int. Cl.: C07C 41/30

(54) **VERFAHREN ZUR HERSTELLUNG VON 2,2'-DIHYDROXY-3,3'-DI-TERT-BUTYL-5,5'-DIMETHOXY-1,1'-BIPHENOL**

(71) Anmelder: Evonik Degussa GmbH, 45128 Essen (DE)
(72) Erfinder: DYBALLA, Katrin Marie, 45657 Recklinghausen (DE); CHRISTIANSEN, Andrea, 18119 Rostock (DE); FRANKE, Robert, 45772 Marl (DE); FRIDAG, Dirk, 45721 Haltern am See (DE); KREIDLER, Burkard, 45219 Essen (DE)

(57) **Zusammenfassung**

Verfahren zur Herstellung von 2,2'-Dihydroxy-3,3'-di-*tert*.-butyl-5,5'-dimethoxy-1,1'-biphenol.

## Beschreibung

Die vorliegende Erfindung betrifft ein neues Verfahren zur Herstellung von 2,2'-Dihydroxy-3,3'-di-*tert*-butyl-5,5'-dimethoxy-1,1'-biphenol durch oxidative Kupplung von 3-*tert*.-Butyl-4-hydroxyanisol (3-BHA).

Symmetrische Biphenole sind für industrielle Anwendungen von großem Interesse (vgl. WO 2005/042547). Diese finden vor allem als Ligandkomponenten für Katalysatoren Anwendung. Dabei kann das Biphenol beispielsweise als Ligandbaustein in der enantioselektiven Katalyse verwendet werden (vgl. Y. Chen, S. Yekta, A. K. Yudin, Chem. Rev. 2003, 103, 3155-3211; J. M. Brunel Chem. Rev. 2005, 105, 857-898; S. Kobayashi, Y. Mori, J. S. Fossey, Chem. Rev. 2011, 11, 2626-2704).

Die direkte Kupplung von Phenolen zu den entsprechenden Biphenolderivaten stellt jedoch nach wie vor eine Herausforderung dar, da diese Reaktionen oftmals weder regio- noch chemoselektiv sind.

In der Literatur sind eine Vielzahl von potentiellen Oxidationsmitteln beschrieben, die in der Lage sind, Phenole, insbesondere *o,p*-disubstituierte Phenole, zu 2,2'-Dihydroxybiphenolen zu kuppeln. Hierbei spielen vor allem Kaliumhexacyanoferrat (III), K₃[Fe(CN)₆ (siehe: a) Adv. Synth. Catal. 2004, 346, 993-1003; b) J. Org. Chem. 2011, 76, 8376-8385) und Natriumperoxodisulfateine entscheidende Rolle.

Die beiden vorgenannten Oxidationsmittel sind jedoch relativ teuer, was sich bei einer großtechnischen Synthese sofort auf den Gesamtpreis des Produktes auswirkt. Zudem fallen bei Verwendung von Kaliumhexacyanoferrat (III) sowie bei Verwendung von Natriumperoxodisulfat unerwünschte Salze und im ersten Fall möglicherweise sogar toxische Cyanide als Nebenprodukte an. Es wird also eine Vielzahl an Nebenprodukten gebildet, deren Abtrennung vom gewünschten Zielprodukt sowie deren Entsorgung aus ökologischer und ökonomischer Sicht verbesserungswürdig ist.

Alternativ lassen sich die Biphenole auch elektrochemisch herstellen (siehe: M. Malkowsky, U. Griesbach, H. Pütter, S. R. Waldvogel, Novel Template-directed Anodic Phenol Coupling Reaction, Chem. Eur. J. 2006, 12, 7482-7488). In dem dort beschriebenen Verfahren wird BHA-Boronsäure in Acetonitril elektrochemisch umgesetzt, um das gewünschte Biphenol zu erhalten. In diesem Verfahren wird somit ein BHA-Borat benötigt, was einen zusätzlichen Syntheseschritt und unnötige Boratabfälle verursacht.

Ein weiteres Verfahren verwendet den kostengünstigen Luftsauerstoff. Die Reaktion läuft in wässriger NaOH ab, während Luft bei 80 °C in das Gemisch geblasen wird (siehe: a) US 4.717.775 (UCC); b) J. Organomet. Chem. 1994, 471, 201.; c) J. Org. Chem. 1989, 54, 4115-4217). Bei der Handhabung mit Luftsauerstoff bzw. ggf. reinem Sauerstoff in einer großtechnischen Produktion sind zusätzliche Vorsichtsmaßnahmen einzuhalten, was in der Regel auch wieder zu einer Verteuerung des Verfahrens führt.

Auch die Reaktion unter Verwendung von Kupfer (II), z.B. Kupferchlorid / TMEDA ist beschrieben (J. Mol. Cat., 1983, 83, 17). Die Reaktion wird in Methanol ohne basischen Zusatz durchgeführt. Die Reaktion kann bei Raumtemperatur durchgeführt werden, jedoch mit sehr langen Reaktionszeiten. Nachteilig ist hier die Verwendung von Metallreagenzien, die ggf. im Produkt stören könnten (beispielsweise bei weiteren Umsetzungen).

Wasserstoffperoxid ist das zweitgünstigste Oxidationsmittel (nach Luftsauerstoff) und reagiert viel schneller mit BHA als Luft. Normalerweise dauert die H₂O₂-Zudosierung ein bis zwei Stunden und nach weiteren 30 Minuten ist die Reaktion beendet. Die Reaktion läuft in wässriger NaOH unter Verwendung von Natriumlaurylsulfat (Natriumdodecylsulfat) als ein kostengünstiges und effizientes Phasentransfermittel ab. Diese Reaktion wurde 1981 von Ciba beschrieben (vgl. EP 35965 B1).
Die vorgenannte europäische Patentschrift offenbart ein Verfahren zur Herstellung von 2,2'-Dihydroxybiphenyl-Verbindungen, wobei als Edukt im Gegensatz zur vorliegenden Erfindung nicht 3-*tert*.-Butyl-4-hydroxyanisol (3-BHA) eingesetzt wird. Stattdessen werden in der EP 35965 B1 nur Phenole der allgemeinen Formel 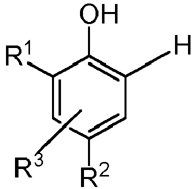 als Edukt eingesetzt, die in para-Stellung zu der phenolischen Hydroxylgruppe Wasserstoff, C₁-C₁₈-Alkyl, C₂-C₆ Alkenyl, ggf. mit C₁-C₄-Alkylresten substituiertes C₅-C₇-Cycloalkyl, Phenyl oder C₇-C₉-Phenylalkyl als Substituenten R² aufweisen.
Lediglich für den Fall, dass die Substituenten R¹ und R³ zusammen einen an die 3,4-beziehungsweise 3',4'-Position gebundenen Butadi-1,3-enylen-1,4-Rest bedeuten, kann R² eine Heteroatome aufweisende Gruppe -(CH₂)ₙCOOR⁴ sein, wobei R⁴ C₁-C₁₈-Alkyl und n = 0, 1 oder 2 ist. In allen anderen Fällen werden ausnahmslos Kohlenwasserstoffe als mögliche Substituenten für R² beschrieben und es wird auch kein Hinweis gegeben, dass diese durch Reste mit einem Heteroatom substituierbar sein könnten. Insbesondere für den Fall, in dem R¹ ein Alkylrest, z.B. ein *tert*.-Butylrest ist, werden als mögliche Substituenten für R² ausschließlich Kohlenwasserstoffe angegeben.

Aufgabe der vorliegenden Erfindung war es nun, ein Verfahren zur Herstellung von 2,2'-Dihydroxy-3,3'-di-*tert*.-butyl-5,5'-dimethoxy-1,1'-biphenol bereitzustellen, das die im Zusammenhang mit dem Stand der Technik beschriebenen Nachteile nicht aufweist. Eine bevorzugte Aufgabe lag weiterhin in der selektiven Herstellung von 2,2'-Dihydroxy-3,3'-di-*tert*.-butyl-5,5'-dimethoxy-1,1'-biphenol, bei der möglichst wenig Nebenprodukte anfallen.

Gelöst wird diese Aufgabe durch ein Verfahren gemäß Anspruch 1.

Verfahren zur Herstellung von 2,2'-Dihydroxy-3,3'-di-*tert*.-butyl-5,5'-dimethoxy-1,1'-biphenol durch oxidative Kupplung von 3-*tert*.-Butyl-4-hydroxyanisol, umfassend die Verfahrensschritte:
a) Erwärmen einer Mischung, umfassend zumindest ein Lösungsmittel, eine anorganische Base und 3-*tert*.-Butyl-4-hydroxyanisol, auf eine Temperatur oberhalb der Raumtemperatur,
b) Zugabe einer Wasserstoffperoxid-Lösung zu der Mischung,
c) Abtrennen des Produkts 2,2'-Dihydroxy-3,3'-di-*tert*.-butyl-5,5'-dimethoxy-1,1'-biphenol.

Die oxidative Kupplung von 3-*tert*.-Butyl-4-hydroxyanisol (3-BHA) zu 2,2'-Dihydroxy-3,3'-di-*tert*.-butyl-5,5'-dimethoxy-1,1'-biphenol (**1**) läuft gemäß Gleichung 1 (GI. 1) ab:

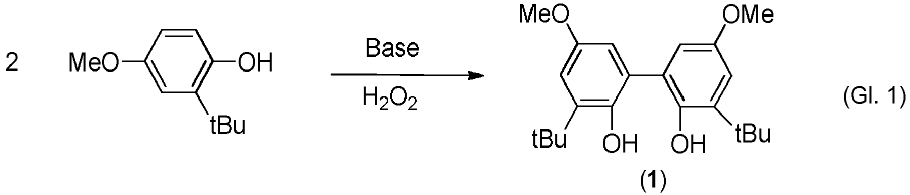

Da es sich bei dem Edukt 3-*tert*.-Butyl-4-hydroxyanisol (3-BHA) um ein *o*,*p*-disubstituiertes Phenol handelt, gibt es nur eine sterisch ungehinderte Stellung zur Rekombination zweier Edukt-Moleküle, die in den meisten Fällen zu dem als Reaktionsprodukt in GI. 1 aufgeführten Homokupplungsprodukt führt.

Aufgrund der partiellen Radikaldichte am Phenolsauerstoff kommt es jedoch auch in geringem Umfang zu einer C-O-Kupplung, die zu dem Biphenylether (**2**) reagiert, welcher in dem Reaktionsprodukt zu < 3 % als Verunreinigung festgestellt werden kann

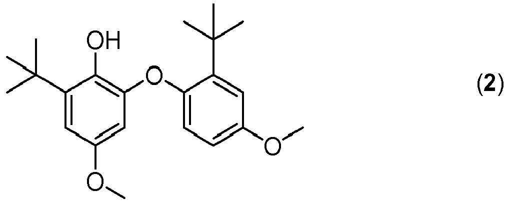

Gemäß einer Ausführungsform des erfindungsgemäßen Verfahrens liegt das eingesetzte Edukt 3-*tert*.-Butyl-4-hydroxyanisol (3-BHA) in einer Isomerenmischung vor, die neben 3-*tert*.-Butyl-4-hydroxyanisol (3-BHA) auch 2-*tert*.-Butyl-4-hydroxyanisol (2-BHA) enthält. Es wurde gefunden, dass das Isomer 2-*tert*.-Butyl-4-hydroxyanisol (2-BHA) im Wesentlichen nicht umgesetzt wird, wohingegen das Isomer 3-*tert*.-Butyl-4-hydroxyanisol (3-BHA) quantitativ gemäß GI. 1 umgesetzt wird.

In einer Ausführungsform des erfindungsgemäßen Verfahrens ist das in der Mischung enthaltene zumindest eine Lösungsmittel ausgewählt aus Wasser, Alkoholen, Kohlenwasserstoffen, Amiden. Ein besonders bevorzugtes Lösungsmittel ist Wasser.

Die in der das Lösungsmittel und 3-*tert*.-Butyl-4-hydroxyanisol (3-BHA) umfassende Mischung enthaltene anorganische Base ist gemäß einer bevorzugten Ausführungsform ein Alkalihydroxid, ein Erdalkalihydroxid, ein Alkalicarbonat oder ein Erdalkalicarbonat, wobei Natriumhydroxid und Kaliumhydroxid besonders bevorzugte anorganische Basen sind. Bezogen auf 1 Äquivalent 3-BHA werden bevorzugt 1 bis 3 Äquivalente, besonders bevorzugt 1,5 bis 2,5 Äquivalente, ganz besonders bevorzugt 1,8 bis 2,2 Äquivalente der anorganischen Base eingesetzt.

Gemäß einer Ausführungsform des Verfahrens enthält die Mischung, die das zumindest eine Lösungsmittel, die anorganische Base und 3-*tert*.-Butyl-4-hydroxyanisol (3-BHA) enthält, außerdem auch eine oberflächenaktive Verbindung. Eine bevorzugte oberflächenaktive Verbindung, die in dem erfindungsgemäßen Verfahren einsetzbar ist, ist Natriumdodecylsulfat (Natriumlaurylsulfat). Die oberflächenaktive Verbindung kann bereits in sehr geringen Mengen eingesetzt werden, beispielsweise etwa 0,0001 bis 0,2 Äquivalente bezogen auf 1 Äquivalent 3-BHA. Bevorzugt ist der Einsatz von 0,05 Äquivalenten oberflächenaktive Substanz bezogen auf 1 Äquivalent 3-BHA.

Erfindungsgemäß wird die Mischung umfassend das zumindest eine Lösungsmittel, die anorganische Base und 3-*tert*.-Butyl-4-hydroxyanisol (3-BHA) auf eine Temperatur oberhalb der Raumtemperatur erwärmt. Beispielsweise wird die Mischung auf eine Temperatur zwischen 30 °C und 100 °C, insbesondere auf eine Temperatur zwischen 75 °C und 95 °C und besonders bevorzugt auf eine Temperatur zwischen 80 °C und 90 °C erwärmt.

In einer Ausführungsform wird die erwärmte Mischung für eine Dauer von mindestens 15 Minuten bei erhöhter Temperatur gerührt, beispielsweise bei einer Temperatur zwischen 75 °C und 95 °C, bevor die Wasserstoffperoxid-Lösung hinzugegeben wird. Bevorzugt wird die Mischung vor Zugabe der Wasserstoffperoxid-Lösung für etwa 30 Minuten bei der erhöhten Temperatur gerührt.

Die Wasserstoffperoxid-Lösung, die zu der Mischung, die das zumindest eine Lösungsmittel, die anorganische Base und 3-*tert*.-Butyl-4-hydroxyanisol (3-BHA) enthält, gegeben wird, ist bevorzugt eine wässrige, beispielsweise eine 30-35 %ige Wasserstoffperoxid-Lösung. Bevorzugt wird pro Äquivalent 3-BHA, das in der Mischung enthalten ist, ein Äquivalent der Wasserstoffperoxid-Lösung zu der Mischung gegeben.

In einer Ausführungsform des Verfahrens erfolgt die Zugabe der Wasserstoffperoxid-Lösung zu der Mischung bei einer Temperatur zwischen 85 °C und 90 °C. Optional wird nach der Zugabe der Wasserstoffperoxid-Lösung für eine Dauer von mindestens 15 Minuten, insbesondere für eine Dauer von etwa 30 Minuten, bei einer Temperatur zwischen 80 °C und 90 °C gerührt.

Die Abtrennung des Produkts 2,2'-Dihydroxy-3,3'-di-*tert*.-butyl-5,5'-dimethoxy-1,1'-biphenol erfolgt vorzugsweise bei Raumtemperatur. Gemäß einer Ausführungsform kann das Produkt dabei mittels einer Zentrifuge und/oder einer Drucknutsche aus der Lösung abgetrennt werden.

Optional wird das Produkt anschließend getrocknet, insbesondere bei erhöhter Temperatur und/oder unter vermindertem Druck, vorzugsweise bis ein Wassergehalt von < 0,1 % erreicht wird.

Das Produkt kann so mit ca. 85 % Ausbeute (bezogen auf den Gesamt-BHA-Gehalt bei Verwendung eines Isomerengemisches als Edukt) und Reinheiten von > 97 % erhalten werden, wobei der Biphenylether **2** die Hauptverunreinigung darstellt. Spuren von monomeren 2-BHA und 3-BHA können ebenfalls vorhanden sein. Betrachtet man nur das reaktive 3-BHA, erhält man sogar quantitative Ausbeuten.

Im Folgenden soll die Erfindung anhand eines Ausführungsbeispiels näher erläutert werden.

### Allgemeine Arbeitsvorschriften

Die Charakterisierung der Produkte erfolgte mittels NMR-Spektroskopie. Chemische Verschiebungen (δ) werden in ppm angegeben. Die Aufnahme von Kernresonanzspektren erfolgte an Bruker Avance 300 bzw. Bruker Avance 400, die gaschromatografische Analyse an Agilent GC 7890A, die Elementaranalyse an Leco TruSpec CHNS und Varian ICP-OES 715, und die ESI-TOF Massenspektrometrie an Thermo Electron Finnigan MAT 95-XP und Agilent 6890 N/5973 Geräten.

### Beispiel 1: Darstellung von 2,2'-Dihydroxy-3,3'-di-tert.-butyl-5,5'-dimethoxy-1,1'-biphenol

Im einem Kolben werden 1360 mL Wasser vorlegt. Unter Rühren werden 6 g Natrium-n-dodecylsulfat, 320 g Natriumhydroxid und 721 g 3-*tert*.-Butyl-4-hydroxyanisol (3-BHA) zugeben. Die Mischung wird auf 85 °C erwärmt und 30 Minuten bei 85 °C rühren gelassen. Anschließend werden 240 mL Wasserstoffperoxid-Lösung (35%ig) zugetropft. Während der Zugabe der Wasserstoffperoxid-Lösung wird die Temperatur zwischen 85 °C und 90 °C gehalten. Nach Beendigung der Zugabe wird für weitere 30 Minuten bei 85 °C rühren gelassen. Dann wird auf Raumtemperatur abkühlen gelassen, filtriert und zweimal mit je 200 mL Wasser gewaschen. Das Produkt wird im Vakuum trocknen gelassen.
Ausbeute: 585,1 g (82 %); Reinheit 99%.

## Patentansprüche

1. Verfahren zur Herstellung von 2,2'-Dihydroxy-3,3'-di-*tert*.-butyl-5,5'-dimethoxy-1,1'-biphenol durch oxidative Kupplung von 3-*tert*.-Butyl-4-hydroxyanisol, umfassend die Verfahrensschritte:
a) Erwärmen einer Mischung, umfassend zumindest ein Lösungsmittel, eine anorganische Base und 3-*tert*.-Butyl-4-hydroxyanisol, auf eine Temperatur oberhalb der Raumtemperatur,
b) Zugabe einer Wasserstoffperoxid-Lösung zu der Mischung,
c) Abtrennen des Produkts 2,2'-Dihydroxy-3,3'-di-*tert.*-butyl-5,5'-dimethoxy-1,1'-biphenol.

2. Verfahren nach Anspruch 1,
wobei das zumindest eine Lösungsmittel ausgewählt ist aus Wasser, Alkoholen, Kohlenwasserstoffen, Amiden.

3. Verfahren nach Anspruch 1 oder 2,
wobei die anorganische Base ein Alkalihydroxid, ein Erdalkalihydroxid, ein Alkalicarbonat oder ein Erdalkalicarbonat ist.

4. Verfahren nach Anspruch 3,
wobei die anorganische Base Natriumhydroxid oder Kaliumhydroxid ist.

5. Verfahren nach einem der Ansprüche 1 bis 4,
wobei die Mischung in Schritt a) weiterhin eine oberflächenaktive Verbindung umfasst.

6. Verfahren nach Anspruch 5,
wobei die oberflächenaktive Verbindung Natriumdodecylsulfat ist.

7. Verfahren nach einem der Ansprüche 1 bis 6,
wobei die Mischung in Schritt a) auf eine Temperatur zwischen 75 °C und 95 °C erwärmt wird.

8. Verfahren nach Anspruch 7,
wobei die in Schritt a) erwärmte Mischung für eine Dauer von mindestens 15 Minuten bei der Temperatur zwischen 75 °C und 95 °C gerührt wird, bevor in Schritt b) die Wasserstoffperoxid-Lösung hinzugegeben wird.

9. Verfahren nach einem der Ansprüche 1 bis 8,
wobei die Zugabe der Wasserstoffperoxid-Lösung in Schritt b) bei einer Temperatur zwischen 85 °C und 90 °C erfolgt.

10. Verfahren nach einem der Ansprüche 1 bis 9,
wobei nach Zugabe der Wasserstoffperoxid-Lösung in Schritt b) für eine Dauer von mindestens 15 Minuten bei einer Temperatur zwischen 80 °C und 90 °C gerührt wird.

11. Verfahren nach einem der Ansprüche 1 bis 10,
wobei das Abtrennen des Produkts bei Raumtemperatur erfolgt.

12. Verfahren nach einem der Ansprüche 1 bis 11,
wobei das Abtrennen des Produkts eine Behandlung mittels Zentrifuge und/oder Drucknutsche einschließt.

13. Verfahren nach einem der Ansprüche 1 bis 12,
wobei das 3-*tert*.-Butyl-4-hydroxyanisol als Bestandteil einer Isomerenmischung eingesetzt wird, die neben 3-*tert*.-Butyl-4-hydroxyanisol auch 2-*tert*.-Butyl-4-hydroxyanisol enthält.
